# EUROPEAN PATENT SPECIFICATION

(11) **EP 1 731 101 B1**
(45) Date of publication and mention of the grant of the patent: **13.05.2009**
(21) Application number: 04807705.1
(22) Date of filing: 24.12.2004
(51) Int. Cl.: A61B 8/08, A61B 5/107, A61B 5/20, A61B 5/00, A61B 8/00

(54) **ULTRASONIC URINARY VOLUME SENSOR**
ULTRASCHALLSENSOR ZUR BESTIMMUNG DES URINVOLUMENS
CAPTEUR ULTRASONIQUE POUR MESURER LE VOLUME D'URINE

(30) Priority: 31.03.2004 JP 2004105737
(43) Date of publication of application: 13.12.2006
(73) Proprietor: National Institute of Advanced Industrial Science and Technology, Tokyo 100-8921 (JP); Takeshiba Electric Co., Ltd., Sagamihara-shi, Kanagawa 229-1124 (JP)
(72) Inventor: KODAMA, H., Nat. Inst. of Adv. Ind. Science & Tech, Tsukuba-shi, Ibaraki 3058566 (JP); KUCHINOMACHI, Y., Nat. Inst. of Adv. Ind. S. & T., Tsukuba-shi, Ibaraki 3058566 (JP); YOSHIMURA, Hisashi, Sagamihara-shi, Kanagawa 2291124 (JP)
(74) Representative: Makovski, Priscilla Mary
(86) International application number: PCT/JP2004/019348
(87) International publication number: WO 2005/099582

(56) References cited:
- GB-A- 2 391 625
- JP-A- 07 171 149
- JP-A- 11 206 721
- JP-A- 2000 210 286
- JP-A- 2001 120 545
- JP-A- 2002 034 934
- JP-A- 2003 190 168
- JP-A- 2004 081 632
- US-B1- 6 359 190
- US-B1- 6 406 431
- US-B1- 6 565 512

## Description

### FIELD OF THE INVENTION

The present invention relates to an ultrasonic urinary volume sensor, and specifically to an ultrasonic urinary volume sensor capable of estimating a volume of urine in a bladder accurately corresponding to any particular individuals and/or conditions thereof.

### DESCRIPTION OF THE PRIOR ART

It is essential to employ a method based on the consideration directed to physiological characteristics of the bladder in order to measure a volume of urine in the bladder in a practical and simplified manner by using ultrasonic waves (see, for example, Patent Document 1).

[Patent document No.1] Japanese Patent Publication No. 407171149.
A method of the prior art as disclosed in the above Patent Document No.1 (a urination alarming apparatus equipped with an irradiation angle automatic selection function) has employed a plurality of oscillators operable at different irradiation angles, in which an oscillator having a particular ultrasonic wave irradiation angle for achieving a maximum receiving wave level can be automatically selected, and a distance between an anterior wall and a posterior wall of the bladder which may be detected by the selected oscillator can be taken as an indicator for estimating a urinary volume in the bladder.

A result from a clinical measurement conducted by using a sensor incorporated with the indicator as disclosed in the above-cited Patent Document No.1 shows a characteristic in which the employed indicator (the distance between the anterior wall and the posterior wall of the bladder) represents a change in a bladder volume in a binary form. It has been revealed that although the method is suitable for such a simple application where if a certain bladder volume has been reached or exceeded, an alarm is given, yet the method is not satisfactorily operable for a case requiring a further precise indication of change in the bladder volume.
US 6 359 190 is similar in that it describes a device using several ultrasonic sensors which measure the distance between the anterior and posterior walls of the bladder in order to estimate the volume of the bladder.
JP 2000-210286 also shows a device with several ultrasonic sensors, which uses indicator values as a measure of the volume of the bladder. The indicator value is derived from the sum of the measured distance between the anterior and posterior walls of the bladder multiplied by an ultrasonic wave echo peak from the posterior wall, and divided by the ultrasonic wave receiving sensitivity of the bladder wall.

Looking into the fact that a control of urination is placed under a control of the autonomic nervous system such as sympathetic nerve and parasympathetic nerve and it does not always exhibit constant aspect but exhibits various aspects as seen from a phenomenon by way of example that when one is exposed to cold stimulation and/or placed under mental strains, the micturition desire emerges in a pattern that is different from his/her ordinary urination pattern, there is still a demand for a sensor capable of capturing the precisely detected change in bladder volume in order to provide a carefully controlled management of urination. For meeting this demand, it is required to precisely analyze a property of bladder expansion.

The present invention has been finally and successfully reached after more than 10 year comprehensive study aiming for solving the problem associated with the prior art as disclosed in the above-cited Patent Document and other publications, and an object thereof is to provide an ultrasonic urinary volume sensor capable of estimating a urinary volume in the bladder accurately corresponding to any particular individuals and/or conditions thereof by incorporating a space time series processing method allowing for an accurate estimation of the urinary volume in the bladder in association with the particular individuals and/or conditions thereof.

The present invention provides an ultrasonic urinary volume sensor comprising:
a probe having a plurality of ultrasonic oscillators for oscillating ultrasonic waves towards a wall surface of a bladder, the probe being adapted to be adhesively placed over a body surface in an abdominal section via an ultrasonic wave transmission medium such that a lower end of said probe can be aligned with an upper end of the pubic bone; and
a processing section for detecting and processing reflective echoes of the ultrasonic waves from said wall surface of the bladder, which have been oscillated by said plurality of ultrasonic oscillators of said probe,
in which the plurality of ultrasonic oscillators are to be disposed along the cephalic direction when the probe is adhesively placed over the body surface in the abdominal section,
and in which said processing section has a CPU section adapted to:
   detect an ultrasonic wave echo peak (Pi) of a posterior wall of the bladder from the reflective echoes of the ultrasonic waves from the wall surface of the bladder, which have been oscillated by each one of said ultrasonic oscillators;
   execute a multiplication of the detected ultrasonic wave echo peak (Pi) by a distance (Di) between an anterior wall and the posterior wall of the bladder that can be specified from said ultrasonic wave echo peak (Pi) for each one of said ultrasonic oscillators; and
   execute an addition of respective values from the multiplications to determine a measured indicator value (PD);
   characterised in that the CPU section is adapted to perform an averaging process on a plurality of said measured indicator values (PD), which have been obtained in a time series, in order to calculate an average indicator value and estimate the urinary volume in the bladder based on the average indicator value and a multiplication coefficient corresponding to a difference among individuals based on their anatomical structures and a posture during the measurement.

Conveniently, the CPU excludes from the calculation of the average indicator value the highest and the lowest values among the plurality of the measured indicator values. A moving average process may be further applied to a plurality of said average indicator values.

Furthermore, the processing section may comprise a hardware section in which the hardware section is electrically connected to the plurality of ultrasonic oscillators of the probe and the CPU section, and the hardware section includes a low noise amplifier, an A/D converting circuit, a waveform memory, a timing generating circuit and an ultrasonic oscillator exciting circuit.

### EFFECT OF THE INVENTION

According to the present invention, the ultrasonic urinary volume sensor that has been configured so as to work adaptively in response to each different individual in his/her anatomical structure and/or to postures such as a lie position, a standing position and a sitting position can provide an accurate and reliable estimation of a urinary volume in the bladder.

### BRIEF DESCRIPTION OF THE DRAWINGS

Fig. 1 is a block diagram schematically showing an embodiment of an ultrasonic urinary volume sensor according to the present invention;

Fig. 2 is a schematic diagram showing an ultrasonic oscillator applicable to an ultrasonic urinary sensor of the present invention;

Fig. 3 shows a graphical representation, in which Fig. 3(a) is a graphical representation plotting a set of measurement data and Fig. 3(b) is a graphical representation plotting a set of data from a space time series average processing applied to the measurement data shown in Fig. 3(a); and

Fig. 4 is a block diagram schematically showing another embodiment of the present invention.

### BEST MODE FOR CARRYING OUT THE INVENTION

Preferred embodiments for carrying out an ultrasonic urinary volume sensor (a urinary volume monitor) according to the present invention will now be described with reference to the attached drawings. An ultrasonic urinary volume sensor according to the present invention designates such an ultrasonic urinary volume sensor characterized in incorporating therein a space time series processing method allowing a urinary volume in the bladder to be estimated accurately corresponding to any specific individuals and/or conditions thereof, which can response to various types of change effectively by a predictive processing while looking up a reference for the specific individual, which reference may be obtained through the modeling operation tailored for the specific individual, such as personal fitting, by applying a learning operation to the previous data on a user with additional consideration of a sensor service condition, such as a posture, in order to deal with an effect on a sensor measurement resultant from a change in posture and/or a physical movement during a measurement conducted by the sensor as well as a dispersion in the ultrasonic wave echo data for the bladder resultant from time series characteristics in a bladder wall structure, which will change under the influence of organs, tissues and the like surrounding the bladder.

Any changes in spatial-temporal structure of the bladder wall surface will induce a change in the ultrasonic wave echoes. Dealing with those changes appropriately may be a necessary procedure for ensuring the accuracy in the estimation of the urinary volume of the bladder. The change in the ultrasonic wave echo contains a component representing a spatial change of the bladder reflecting the bladder expansion and a temporal varying component from the effect of the surrounding tissues and organs around the bladder. Processing those components appropriately in accordance with each specific application may allow to obtain desired physiological information of the bladder. The present invention involves the space time series processing directed for measuring a change in the bladder volume reliably, which will be described in more detail.

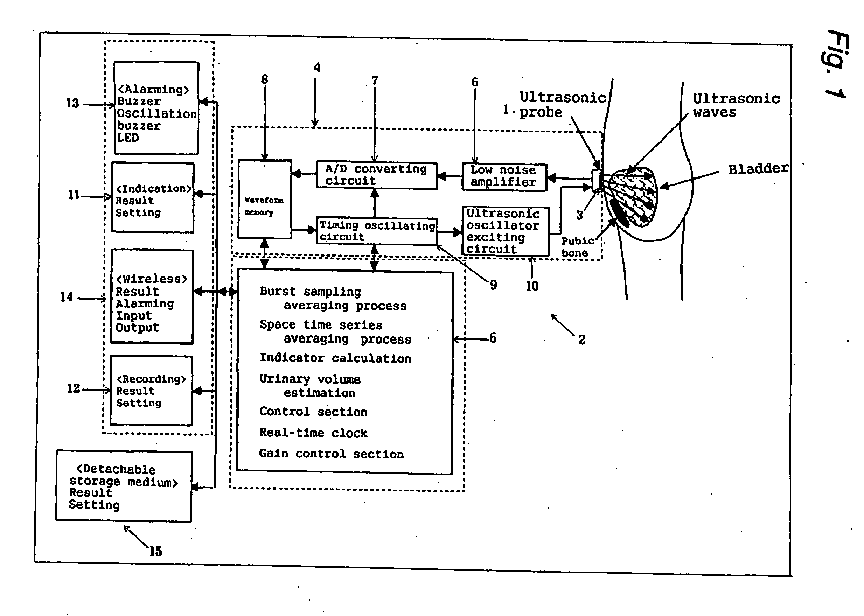

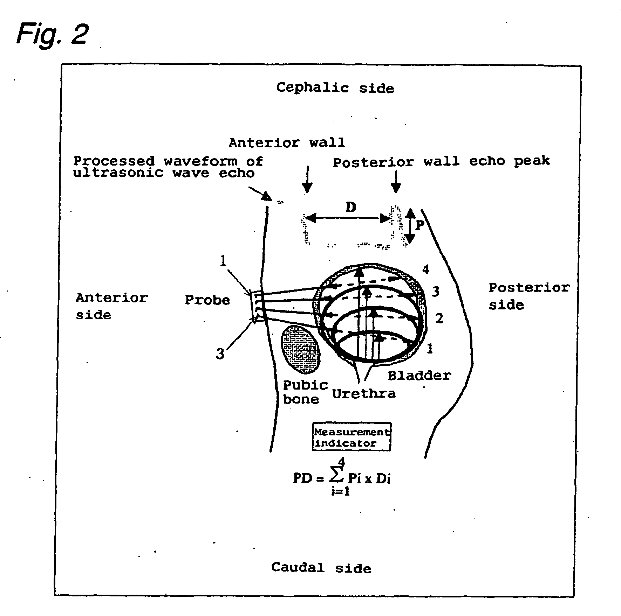

Fig. 1 shows an ultrasonic urinary volume sensor (a urinary volume monitor). This ultrasonic urinary volume sensor comprises primarily a probe (an ultrasonic search unit) 1 and a processing section 2. As shown in Figs. 1 and 2, the probe 1 includes four ultrasonic oscillators 3 for oscillating ultrasonic waves toward a wall surface of the bladder and the probe 1 is adhesively placed over the body surface in the abdominal section such that a lower end of the probe 1 is aligned with a point on the body surface just above an upper end of the pubic bone. As well known to those ordinary skilled in the art, from a reason that if the probe 1 is directly placed over the body surface in the abdominal section, a layer of air between the probe 1 and the body surface in the abdominal section will inhibit an efficient transmission of the ultrasonic waves, the probe 1 is adhesively placed over the body surface in the abdominal section via a ultrasonic wave transmission medium, such as ultrasonic gel, interposed therebetween. The four ultrasonic oscillators 3 are disposed along a direction of the bladder expansion as shown in Fig. 1 (approximately in the vertical direction in Fig. 1). The bladder located within the pelvis exhibits a characteristic expansion manner defined by its anatomical structure. The base of the bladder accretes to a tissue in the base of the pelvis to restricts a motion thereof, and it has been confirmed from the MRI measurement that the bladder expansion in association with the accumulation of the urine takes effect primarily in the cephalic direction, while pushing the relatively movable small intestine aside. Along this expansion direction, the four ultrasonic oscillators 3 are evenly spaced by a predetermined distance therebetween.

The processing section 2 is serving for detecting and processing reflective echoes of the ultrasonic waves from the wall surface of the bladder, which have been oscillated by the set of four ultrasonic oscillators 3 of the probe 1, and the processing section 2 comprises primarily a hardware section (an ultrasonic wave measuring section) 4 and a CPU section 5, as shown in Fig. 1. The hardware section 4 is electrically connected to the four ultrasonic oscillators 3 of the probe 1 and to the CPU 5, and the hardware section 4 includes a low noise amplifier 6, an A/D converting circuit 7, a waveform memory 8, a timing oscillating circuit 9 and an ultrasonic oscillator exciting circuit 10.

The ultrasonic oscillators 3 of the probe 1 adhesively placed over the body surface in the abdominal section are actuated by the timing oscillating circuit 9 and the exciting circuit 10 so as to oscillate the ultrasonic waves toward the wall surface of the bladder. The reflective echoes of the ultrasonic waves from the wall surface of the bladder, which have been oscillated by each of the plurality of ultrasonic oscillators 3, are amplified in the low noise amplifier 6 and then processed by the A/D converting circuit 7 into digital signals, which are in turn stored in the waveform memory 8.

When the bladder expands in association with the accumulation of the urine, firstly a peak of the echo from the posterior wall of the bladder (the reflective echo) emerges in the echo of the ultrasonic waves generated by one of the oscillator 3 oscillating the waves in the direction closer to the base of the bladder, and as the bladder volume increases further, the peaks of the echo from the posterior wall of the bladder are sequentially detected in the echoes of the ultrasonic waves generated by other oscillators 3 directed toward more cephalic direction. Accordingly, if the waveforms of the echoes of the waves generated by respective oscillators 3 are indicated as they are overlapped, the expansion of the bladder can be visually recognized in an easy way with the number of the peaks of the echo from the posterior wall of the bladder increasing sequentially.

The CPU section 5 detects an ultrasonic wave echo peak (P) (a digital signal) of the posterior wall of the bladder from the reflective echoes (digital signals) of the ultrasonic waves from the wall surface of the bladder, which have been oscillated by respective ultrasonic oscillators, and then executes a multiplication of the detected ultrasonic wave echo peak (P) by a distance between the anterior wall and the posterior wall of the bladder (D) specified from the ultrasonic wave echo peak (P) for each one of the ultrasonic oscillators, and thus determine a measured indicator value (PD) by an addition of respective values determined from the multiplication (see Fig. 2). Subsequently, thus determined measured indicator value (PD) is multiplied by a predetermined coefficient corresponding to a difference among individuals based on the anatomical structure and/or a specific posture during the measurement to thereby estimate the urinary volume in the bladder reliably.

To explain specifically, the measured indicator value (PD) can be arithmetically determined by multiplying the distance between the anterior wall and the posterior wall of the bladder (D), which can be specified by the emergence of the ultrasonic wave echo peak (P) of the posterior wall of the bladder, by the magnitude of the echo peak (P), and then by summing up thus obtained values over respective oscillators 3. This arithmetic operation method is based on the waveform processing that has taken practical aspect of emergence of the ultrasonic wave echo waveform of the bladder wall into account. It has been confirmed from the measurement in a clinical examination scene that the quantitative characteristic of thus determined indicator value is sufficiently satisfactory. It may be also possible to deal with the difference among individuals in anatomical structure and the like and/or the specific posture, including a lie position, a standing position and a sitting position, by multiplying the indicator value by an appropriate coefficient (e.g., a predetermined coefficient to make a correction to the difference among individuals and/or the posture during the measurement), and thus to estimate the urinary volume in the bladder accurately.

Although the indicator value increases incrementally in association with the accumulation of the urine in the bladder, some dispersion will be introduced into the value. This dispersion is resultant from a reflecting mechanism of the ultrasonic wave on the wall surface of the bladder and thus the dispersion in itself is unavoidable. Although the indicator values tend to disperse from one measurement time to another measurement time, its trend according to the time series exhibits quite a nice correspondence to the accumulation of the urine, and so applying the appropriate time series data processing may allow a reasonable estimation value of the urinary volume in the bladder to be obtained. Achieving the optimal data processing requires a set of detailed information relating to a factor which may contribute to the spatial-temporal varying characteristics of the wall surface of the bladder. From the viewpoint of the problem in the actual practice, since the optimal data processing in real time is not feasible, a simplified processing method, including the predictive processing, may be used, and consequently the achievement of the optimal data processing requires the selection of the space time series processing method suitable for each particular situation.

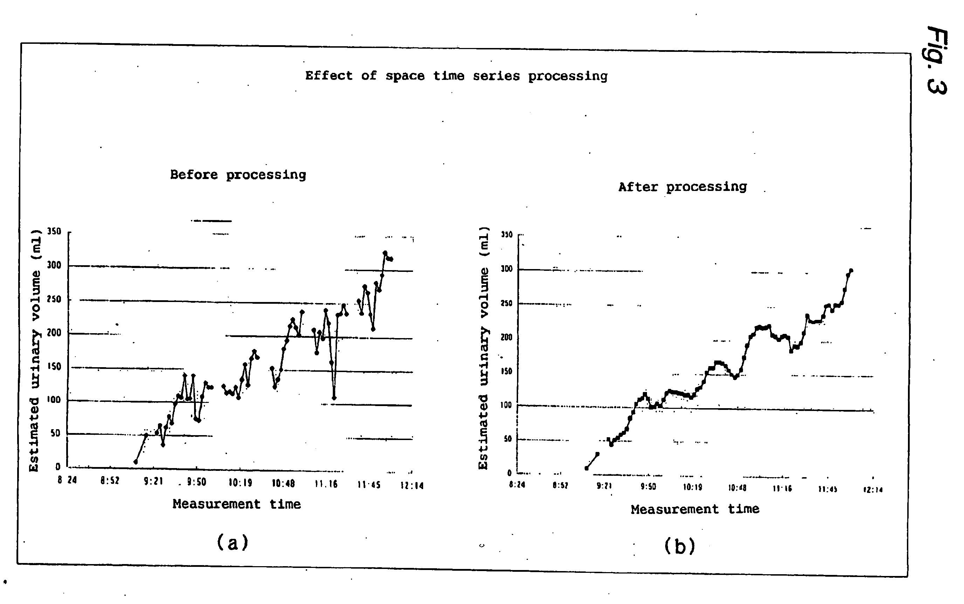

Based on the above-discussed point, in the embodiment of the present invention, the probe 1 incorporated with four of the ultrasonic oscillators 3 aligned in the expansion direction is adhesively placed on the body such that the lower end of the probe 1 is disposed on the upper end of the pubic bone and the measurement is carried out at every one second repeatedly by 10 times (i.e., the measurement over 10 seconds) to determine the indicator values (PD), wherein the highest two values and the lowest two values are excluded from thus determined indicator values and an average over the remaining six values is output. In the actual practice, the above-defined operation were repeated at every two minutes (carried out two or more times in the burst) to conduct an experimental measurement (a burst sampling averaging), and it was recognized from the obtained good result that the method of the embodiment is full of promise as the simplified processing method. Fig. 3(a) shows the data from this experimental measurement. Further, as shown in Fig. 3(b), if the moving average method (the space time series averaging) is further applied to the data shown in Fig. 3(a), the urinary volume estimation of higher precision can be obtained.

An estimation value of the urinary volume (result) obtained in the above-described operation may be indicated in a display section 11 (see Fig. 1) and also recorded in a recording section 12. Further, as shown in Fig. 1, the display section 11 may be used for setting a parameter required for determining the estimation value of the urinary volume and/or a threshold value for alarming, and the sensor may include an alarming section (an alarm output function) consisting of a buzzer, an oscillation buzzer, a LED or the like, as designated by reference numeral 13. It is to be noted that the display section 11, the recording section 12 and the alarming section 13 are electrically connected to the CPU section 5.

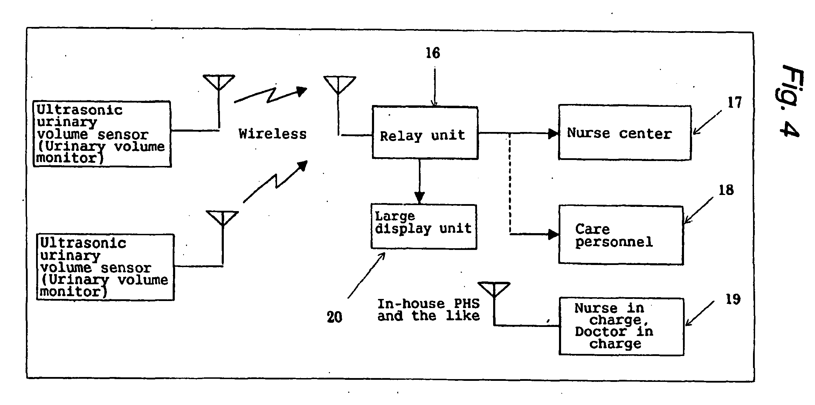

Further, the ultrasonic urinary volume sensor may also include a wireless data communication function as designated by reference numeral 14. This wireless communication function 14 is electrically connected to the CPU 5. In this case, for example, as shown in Fig. 4, it may become possible that the information (signal) originating from the wireless data communication function of the ultrasonic urinary volume sensor according to the present invention can be transmitted to a nurse center 17 through a communication function (a LAN or any dedicated line) provided in a hospital or other facilities via a relay unit 16 or directly, so that the nurse center 17 can be informed of the condition of a patient. Further as designated by reference numeral 18, not only the nurse center but also a care personnel for the patient can be informed of the condition of the patient. Further, as designated by reference numeral 19, the information can be given to a doctor and/or nurse in charge quickly without any delay over a transmission means, such as a in-house PHS and the like. In addition, as designated by reference numeral 20, the information can be transmitted via the relay unit to a large display unit where the information may be presented. In this way, the ultrasonic urinary volume sensor is adapted to inform any authorized people, such as the doctor, nurse and care personnel, of the condition of the patient quickly without any delay by including the wireless communication function in the sensor.

The ultrasonic urinary volume sensor may comprise a detachable storage medium 15. The storage medium 15 is electrically connected to the CPU section 5, and information signals representing a urinary volume estimation value (result), an ultrasonic waveform, an operation result and the like are sent from the CPU section 5 to the storage medium 15 and stored therein. The data stored in the detachable storage medium 15 may be reproduced in a personal computer, for example, and may be used in giving a diagnosis.

In addition, as shown in Fig. 1, the CPU section 5 may comprise a real-time clock serving for outputting a signal at each predetermined timing. The CPU section 5 controls the hardware section 4 based on the signals output from the real-time clock to allow the measurement to be carried out at regular intervals. The ultrasonic urinary volume sensor may be powered on in response to the signal output from the real-time clock and powered off after a predetermined time elapse following the end of the measurement. This arrangement can save the electric power and help achieve low power consumption.

Further, as shown in Fig. 1, the CPU section 5 may comprise a gain control section serving for controlling a gain of the low noise amplifier 6. This gain control section may provide an automatic control of an amplification factor (e.g., AGC) of the low noise amplifier 6.

The probe 30 may further comprise a triaxial acceleration sensor (not shown). This allows for an automatic recognition of the posture of a patient (the position of the patient relative to the axis of earth), such as a standing position, a sitting position and a lie position, and thus an automatic selection of the algorithm for correcting operation of the estimated urinary volume value, to thereby enhance the measurement accuracy.

Further, it may be also possible to monitor the echoes obtainable from a plurality of ultrasonic oscillators 3 to find any unusual adhesive condition between the ultrasonic oscillators 3 and the body surface from the irregularity of the echoes, and if so, the correction may be applied. Furthermore, if the deviation has been induced in the estimated urinary volume in the bladder due to the physical movement, the correction in consistency with the incremental characteristic of the urinary volume in the bladder may be automatically applied to thereby enhance the accuracy of the estimated urinary volume value. Further, the condition of the echoes of the ultrasonic waves from the posterior wall of the bladder as well as from the organs located behind the bladder may be monitored, so that the micturition desire can be recognized on behalf of the patient. That is, when the urinary volume in the bladder has reached a urination level, the height of the echoes from the posterior wall of the bladder has become high in a short time. This indicates that a reflection efficiency from the bladder has been increased, and at the same time, in conjunction with this increased reflection efficiency, the echo height of the echoes from the organs located behind the posterior wall of the bladder become smaller. Accordingly, the micturition desire can be detected on behalf of the patient from the correlation between respective echoes so that a third person can inform the patient of his/her detected micturition desire

Further, the probe 1 may be adhesively placed over the body surface in the abdominal section of the patient not only occasionally for a measurement but also for all the time. For example, it may be contemplated to use an ultrasonic bonding sheet implementing an ultrasonic wave transmission medium with a two-sided tape to allow for the constant wearing of the probe 1. This may help measure the urinary volume of the patient all the time.

## Claims

1. An ultrasonic urinary volume sensor comprising:
a probe (1) having a plurality of ultrasonic oscillators (3) for oscillating ultrasonic waves towards a wall surface of a bladder, the probe (1) being adapted to be adhesively placed over a body surface in an abdominal section via an ultrasonic wave transmission medium such that a lower end of said probe can be aligned with an upper end of the pubic bone; and
a processing section (2) for detecting and processing reflective echoes of the ultrasonic waves from said wall surface of the bladder, which have been oscillated by said plurality of ultrasonic oscillators (3) of said probe (1),
in which the plurality of ultrasonic oscillators (3) are to be disposed along the cephalic direction when the probe (1) is adhesively placed over the body surface in the abdominal section,
and in which said processing section (2) has a CPU section adapted to:
detect an ultrasonic wave echo peak (Pi) of a posterior wall of the bladder from the reflective echoes of the ultrasonic waves from the wall surface of the bladder, which have been oscillated by each one of said ultrasonic oscillators (3);
execute a multiplication of the detected ultrasonic wave echo peak (Pi) by a distance (Di) between an anterior wall and the posterior wall of the bladder that can be specified from said ultrasonic wave echo peak (Pi) for each one of said ultrasonic oscillators (3); and
execute an addition of respective values from the multiplications to determine a measured indicator value (PD);
**characterised in that** the CPU section is adapted to perform an averaging process on a plurality of said measured indicator values (PD), which have been obtained in a time series, in order to calculate an average indicator value and estimate the urinary volume in the bladder based on the average indicator value and a multiplication coefficient corresponding to a difference among individuals based on their anatomical structures and a posture during the measurement.

2. The ultrasonic urinary volume sensor according to claim 1, wherein the CPU is;
adapted to exclude from the calculation of the average indicator value the highest and the lowest values among the plurality of said measured indicator values (PD).

3. The ultrasonic urinary volume sensor according to claim 2, wherein the CPU is adapted to apply a moving average process to a plurality of said average indicator values.

4. The ultrasonic urinary volume sensor according to claim 1, in which
the processing section comprises a hardware section, wherein
the hardware section is electrically connected to the plurality of ultrasonic oscillators of the probe and the CPU section, and includes a low noise amplifier, an A/D converting circuit, a waveform memory, a timing generating circuit and an ultrasonic oscillator exciting circuit.

## Patentansprüche

1. Ultraschallsensor zur Bestimmung des Urinvolumens
mit einer Sonde (1) mit mehreren Ultraschall-Oszillatoren (3) zum Aussenden von Ultraschallwellen gegen eine Wandungsoberfläche einer Blase hin, wobei die Sonde (1) klebend auf einer Körperoberfläche im Bauchbereich über ein Übertragungsmedium für Ultraschallwellen derart anbringbar ist, dass das untere Ende der Sonde im Bereich des oberen Endes des Schambeins liegt;
und mit einem Verarbeitungsteil (2) zum Detektieren und Verarbeiten von Echos der Ultraschallwellen, die von der Wandungsoberfläche der Blase reflektiert worden sind, die von den mehreren Ultraschall-Oszillatoren (3) der Sonde (1) angestrahlt worden ist,
wobei die mehreren Ultraschall-Oszillatoren (3) entlang der Kopfrichtung angeordnet sind, wenn die Sonde (1) klebend auf der Körperoberfläche im Bauchbereich angeordnet sind,
und wobei der Verarbeitungsteil (2) einen CPU-Teil umfasst, mittels dessen:
ein Ultraschallwellen-Echo-Peak (Pi) einer rückwärtigen Wandung der Blase aus den Reflexionsechos der Ultraschallwellen von der rückwärtigen Wandung der Blase detektierbar ist, die von dem jeweiligen Ultraschall-Oszillator (3) angestrahlt worden ist;
eine Multiplikation des detektierten Ultraschallwellen-Echo-Peaks (Pi) mit einer Distanz (Di) zwischen einer vorderen Wandung und einer rückwärtigen Wandung der Blase ausführbar ist, die aus dem Ultraschallwellen-Echo-Peak (Pi) des jeweiligen Ultraschall-Oszillators (3) spezifizierbar ist;
und eine Addition der jeweiligen Werte aus den Multiplikationen durchführbar ist, um einen gemessenen Indikatorwert (PD) zu bestimmen;
**dadurch gekennzeichnet, dass** in dem CPU-Teil ein Mittelungsprozess über mehrere gemessene Indikatorwerte (PD) ausführbar ist, die in einer zeitlichen Reihenfolge erhalten worden sind, um einen mittleren Indikatorwert zu berechnen und das Urinvolumen in der Blase aufgrund eines mittleren Indikatorwertes und eines Multiplikationskoeffizienten zu berechnen, der auf dem Unterschied zwischen individuellen Personen aufgrund ihrer anatomischen Struktur und ihrer Lage während der Messung beruht.

2. Ultraschall-Sensor zur Bestimmung des Urinvolumens nach Anspruch 1, bei dem die CPU in der Lage ist, aus der Kalkulation des Indikatormittelwertes die höchsten und niedrigsten Werte der gemessenen Indikatorwerte (PD) auszuschließen.

3. Ultraschallsensor zur Bestimmung des Urinvolumens nach Anspruch 2, bei dem mittels der CPU auf mehrere Indikator-Mittelwerte ein beweglicher Mittelungsprozess ausführbar ist.

4. Ultraschallsensor zur Bestimmung des Urinvolumens nach Anspruch 1, bei dem der Verarbeitungsteil einen Hardwareteil, der elektrisch mit den mehreren Ultraschall-Oszillatoren der Sonde und dem CPU-Teil verbunden ist, und einen geräuscharmen Verstärker, einen A/D-Wandler, einen Wellenformspeicher, einen Taktgenerator und einen Anregungskreis für einen Ultraschall-Oszillator umfasst.

## Revendications

1. Capteur ultrasonique pour mesurer le volume d'urine comprenant :
une sonde (1) ayant une pluralité d'oscillateurs ultrasoniques (3) pour faire osciller des ondes ultrasonores vers une surface de paroi d'une vessie, la sonde (1) étant adaptée pour être placée de manière adhésive sur une surface du corps dans une section abdominale grâce à un milieu de transmission d'ondes ultrasonores de sorte qu'une extrémité inférieure de ladite sonde puisse être alignée avec une extrémité supérieure du pubis ; et
une section de traitement (2) pour détecter et traiter des échos de réflexion des ondes ultrasonores provenant de ladite surface de paroi de la vessie, qu'on a fait osciller par ladite pluralité d'oscillateurs ultrasoniques (3) de ladite sonde (1),
dans lequel la pluralité d'oscillateurs ultrasoniques (3) doivent être disposés le long de la direction céphalique quand la sonde (1) est placée de manière adhésive sur la surface du corps dans la section abdominale,
et dans lequel ladite section de traitement (2) possède une section d'unité centrale adaptée pour :
détecter un pic d'écho d'ondes ultrasonores (Pi) d'une paroi postérieure de la vessie à partir des échos de réflexion des ondes ultrasonores provenant de la surface de paroi de la vessie, qu'on a fait osciller par chacun desdits oscillateurs ultrasoniques (3) ;
exécuter une multiplication du pic d'écho d'ondes ultrasonores détectées (Pi) par une distance (Di) entre une paroi antérieure et la paroi postérieure de la vessie qui peut être spécifiée par ledit pic d'écho d'ondes ultrasonores (Pi) pour chacun desdits oscillateurs ultrasoniques (3) ; et
exécuter une addition des valeurs respectives provenant des multiplications pour déterminer une valeur d'indicateur mesurée (PD) ;
**caractérisé en ce que** la section d'unité centrale est adaptée pour réaliser un traitement moyen sur une pluralités desdites valeurs d'indicateur mesurées (PD), qui ont été obtenues dans une série chronologique, afin de calculer une valeur d'indicateur moyenne et d'estimer le volume urinaire dans la vessie par rapport à la valeur d'indicateur moyenne et un coefficient de multiplication correspondant à une différence parmi des individus selon leur structure anatomique et une posture durant la mesure.

2. Capteur ultrasonique pour mesurer le volume d'urine selon la revendication 1, dans lequel l'unité centrale est :
adaptée pour exclure du calcul de la valeur d'indicateur moyenne les valeurs les plus élevées et les plus basses parmi la pluralité desdites valeurs d'indicateur mesurées (PD).

3. Capteur ultrasonique pour mesurer le volume d'urine selon la revendication 2, dans lequel l'unité centrale est adaptée pour appliquer un traitement moyen mobile à une pluralité desdites valeurs d'indicateur moyennes.

4. Capteur ultrasonique pour mesurer le volume d'urine selon la revendication 1, dans lequel la section de traitement comprend une section matérielle, où la section matérielle est reliée de manière électrique à la pluralité d'oscillateurs ultrasoniques de la sonde et de la section d'unité centrale, et comprend un amplificateur à faible bruit, un circuit de conversion analogique/numérique, une mémoire de forme d'ondes, un circuit de synchronisation et un circuit d'excitation d'oscillateurs ultrasoniques.
